# EUROPEAN PATENT APPLICATION

(11) **EP 3 613 400 A1**
(43) Date of publication of application: **26.02.2020**
(21) Application number: 18788232.9
(22) Date of filing: 19.04.2018
(51) Int. Cl.: A61J 1/20, A61M 5/14, A61J 1/00

(54) **DILUTION AND DRIP CHAMBER**

(30) Priority: 20.04.2017 BR 102017008203
(71) Applicant: Caetano, Norival, 05616-010 Sao Paulo (BR)
(72) Inventor: Caetano, Norival, 05616-010 Sao Paulo (BR)
(74) Representative: Sarpi, Maurizio
(86) International application number: PCT/BR2018/050118
(87) International publication number: WO 2018/191804

(57) **Abstract**

Dilution and drip chamber for the preparation and administration of injectable solutions for use with a plastic bag (31) with open-and-close valve (33), infusion equipment (32) and syringe (28) with male luer-lock terminal (8); the chamber (30) comprises semi-rigid and transparent plastic tube sector (12), closed by top (16) and bottom (17) covers; on the wall surface (18) of the cover (16) there is an air filter (19) and tubular extension (35) with terminal (8) and cover (34); the tube sector (12) has volumetric scale (14); the bottom cover (17) have tubular extension (37) starting from the base wall (25) in which a conical base (46) of the valve (33) and female luer-lock terminal (45) is inserted; in the upper portion (42) of the outer body (41) of the cover (34) there are radial ribs (43); from the center of the cover (34) a hollow plastic tube (44) goes beyond the base of the cover (34), ending as terminal (45).

## Description

### FIELD OF APPLICATION

This specification is an Invention Patent application proposing a dilution and drip chamber for preparation and administration procedures of injectable pharmaceutical solutions, thus integrating the universe of so-called hospital devices.

### INTRODUCTION

This Invention Patent application describes an innovative dilution and drip chamber for use in conjunction with an open-and-close mechanism, luer-lock infusion set and syringe with luer-lock terminal without needle. This set, which is now incorporated into this dilution and drip chamber, represents the modern approach for preparing and administering injectable solutions, revolutionizing the procedures involved. The dilution and drip chamber, which is the object of this Invention Patent application, respects the same operating concept, complementing this fast and safe set of devices for the preparation and administration procedures for injectable pharmaceutical solutions. This dilution and drip chamber, object of this Invention Patent application, supplants in terms of ease of use and safety that normally attributed to a particular type of hospital use device, belonging to the state of the art known as "equipment with burette", which is used for intermittent administration of pharmaceutical solutions by intravenous infusion (drop by drop into a vein).

### STATE OF THE ART

As health professionals know, the injectable pharmaceutical solutions require preparation in clean environments, respecting aseptic techniques, taking care to avoid puncture accidents by metal needles or plastic spikes (plastic perforators coupled to medical devices) and also particle contamination that can detach when puncturing stoppers or membranes in plastic bags that hold injectable solutions.

Through the Invention Patent PI 1003460-9, granted on 01.06.2015 and named *"BOLSA PARA ACONDICIONAMENTO, RECONSTITUIÇÃO E*/*OU DILUIÇÃO DE PRODUTOS INJETÁVEIS* [BAG FOR PACKAGING, RECONSTITUTION AND/OR DILUTION OF INJECTABLE PRODUCTS]", authored by the applicant, it was pointed out all these concerns and, from the project addressed in said patent document, a new plastic intravenous solution bag with open-and-close mechanism was launched, commercially identified by the trademark "NC Bag®" ("No Coring Bag", a bag that does not generate particles), that avoids the use of metal needles and plastic spikes, connecting directly to the syringe luer-lock terminal and also using an infusion equipment with luer-lock terminal, specially developed for coupling to this bag.

From this first Invention Patent, the applicant herein has improved the open-and-close mechanism of this bag to allow, if needed, repeated opening and closing operations of this mechanism without solution leakage. This has resulted in developments in the Invention Patent application BR 102016 016091 0, filed in 07.11.2016 and named "*VÁLVULA COM MECANISMO ABRE-E-FECHA PARA BOLSA UTILIZADA PARA ACONDICIONAMENTO*, *RECONSTITUIÇÃO E*/*OU DILUIÇÃO DE PRODUTOS INJETÁVEIS* [VALVE WITH OPEN-AND-CLOSE MECHANISM FOR BAG USED FOR PACKAGING, RECONSTITUTION AND/OR DILUTION OF INJECTABLE PRODUCTS]".

Based on these documents (Invention Patent and Invention Patent application), the applicant is continuing to develop new products, always focused on the practicality of use and the safety of the professional and the patient.

The dilution and drip chamber, object of this Invention Patent application, was only conceivable by incorporating the concepts and devices developed in the aforementioned patent ("BOLSA PARA ACONDICIONAMENTO, RECONSTITUIÇÃO E/OU DILUIÇÃO DE PRODUTOS INJETÁVEIS") and also in the enhancement that prompted the Invention Patent application BR 102016 016091 0 ("VÁLVULA COM MECANISMO ABRE-E-FECHA PARA BOLSA UTILIZADA PARA ACONDICIONAMENTO, RECONSTITUIÇÃO E/OU DILUIÇÃO DE PRODUTOS INJETÁVEIS").

The dilution and drip chamber, object of this Invention Patent application, together with the mentioned patented bag and the infusion equipment with luer-lock terminal constitute the evolution that revolutionizes the preparation and administration of injectable solutions.

In order for such an evolution be readily understood, it will be described firstly how the traditional procedure for preparing and administering intravenous infusion solutions is, and then this invention, a dilution and drip chamber, will be presented, and how it complements the NC Bag® set, pouch with open-and-close mechanism, and infusion equipment with luer-lock terminal, as addressed in the granted Invention Patent PI1003460-9 and Invention Patent application BR 10 2016 016091-0.

### TRADITIONAL PROCEDURE

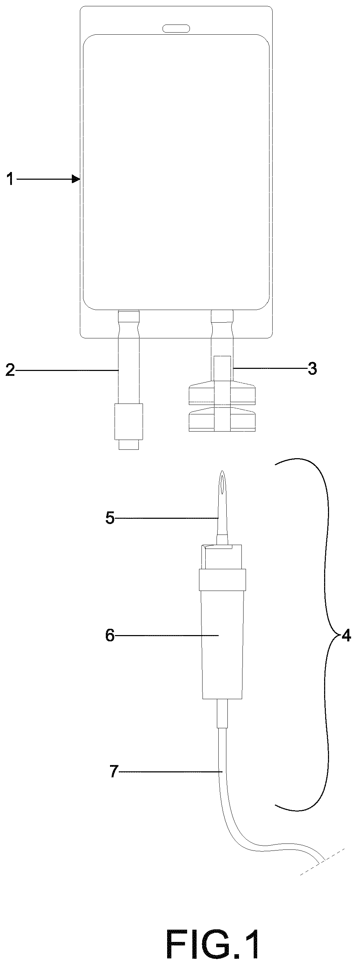

Through figures 1 to 4, the traditional procedure for preparing and administering pharmaceutical solutions by intravenous infusion will be described, using the devices belonging to the prior art (traditional plastic bag for intravenous solutions, traditional infusion equipment, traditional equipment with burette and syringe with metallic needle).

Figure 1 illustrates a schematic overview of a traditional plastic bag for intravenous solutions 1 equipped with two ports or ways, being an inlet port 2, with perforable membrane, and an outlet port 3 also with perforable membrane; figure 1 also illustrates a traditional infusion equipment 4 positioned towards the outlet port 3 of traditional plastic bag for intravenous solutions 1, which has a puncture projection (plastic spike) 5 and a small drip chamber 6, from where a flexible tube sector 7 intended for the patient's vein comes off.

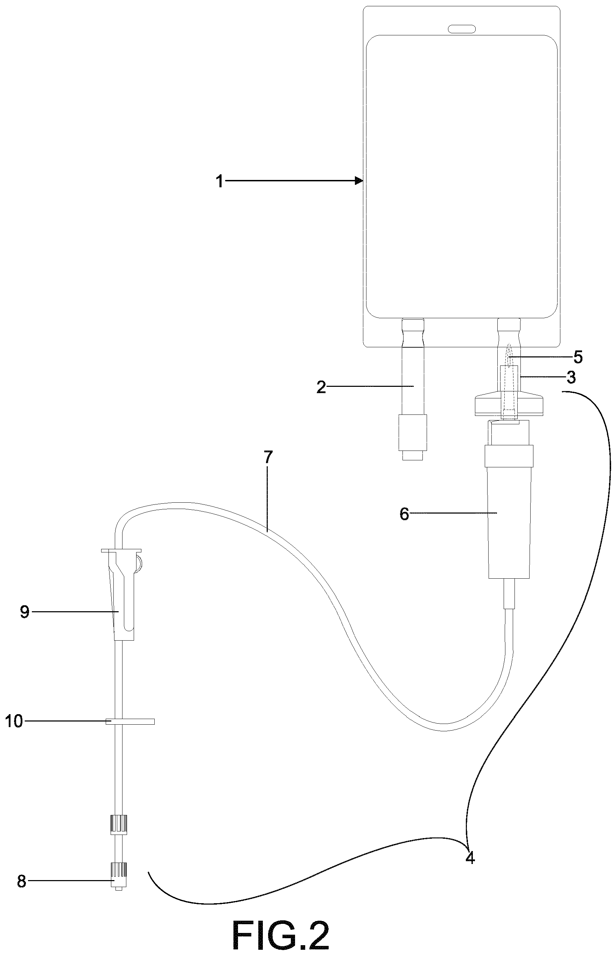

Figure 2 illustrates a schematic overview of the same traditional plastic bag for intravenous solutions 1 with traditional infusion equipment 4 coupled to the outlet port 3. The traditional infusion equipment 4, partially illustrated in figure 1, is fully illustrated in figure 2, and the flexible tube sector 7 has a male luer-lock terminal 8, a "roller clamp"-type device 9 for flow regulation and a plastic clamp 10 to be triggered when full flow closure is desired.

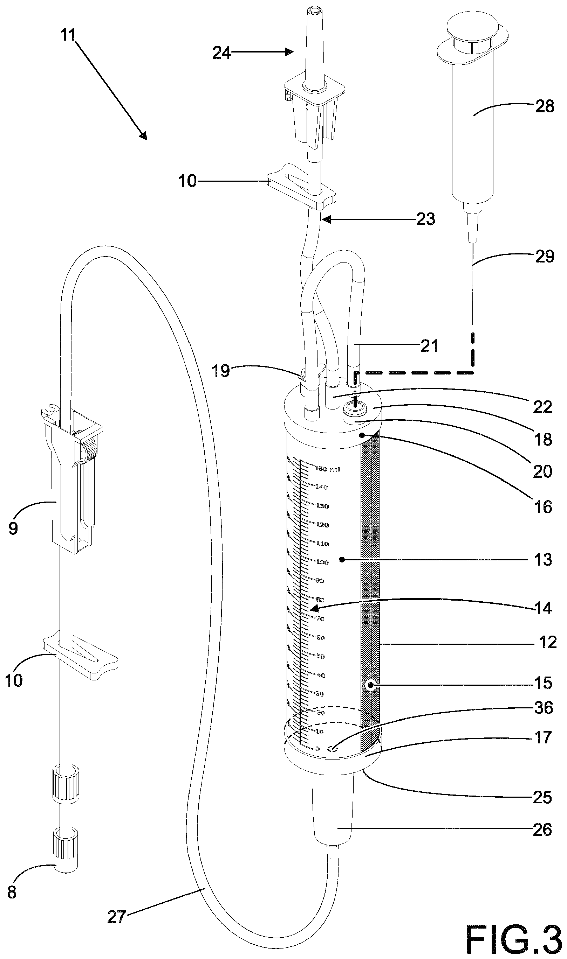

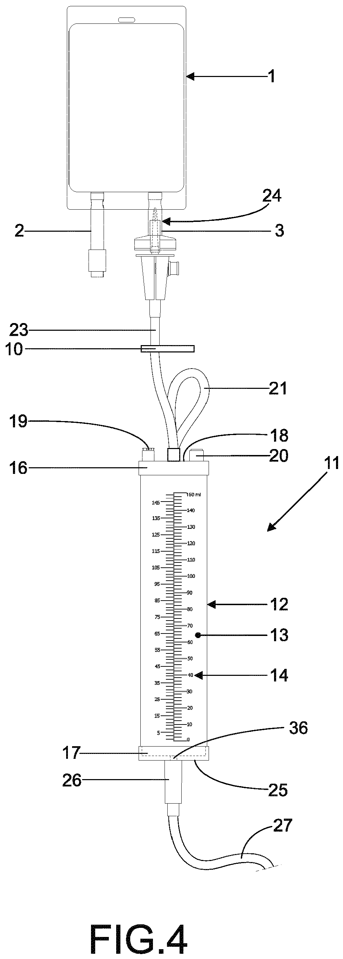

Figure 3 illustrates a perspective view of a traditional equipment with burette 11, having a central component that is a semi-rigid and transparent plastic tube sector (which is the burette) 12, that, on the front portion of its wall 13, presents a graduated volume scale 14 (expressed in ml), while the posterior portion of this wall 13 features white background trim 15, to facilitate visualization of the graduated volume scale 14 and checking any problems in the solution such as, for example, particles. The upper and lower extremes of the semi-rigid and transparent plastic tube sector 12 of the traditional equipment with burette 11 are closed by respective top 16 and bottom 17 caps. On the wall face 18 of the top cover 16 are incorporated: an air inlet port 19 provided with filter and cover; a liquid inlet port with a perforable membrane 20; a handle 21 that allows the traditional equipment with burette 11 can be hung; a connection 22 for a flexible tube sector 23, at the end of which a plastic spike is positioned 24 that will access the intravenous solution contained in a traditional plastic bag for intravenous solutions 1 where the diluent in the desired volume for the product contained in the burette 12 comes from. Throughout this flexible tube sector 23 there is a plastic clamp 10 that, whenever necessary, makes total blockage of fluid flowing from traditional plastic bag for intravenous solutions 1 after the plastic spike is introduced into this bag. The bottom cover 17 of the traditional equipment with burette 11 has a liquid through hole 36 illustrated in dashed line in figures 3 and 4; from the base 25 of this cover comes a small drip chamber 26 for flow of the liquid contained in the burette 12; the small drip chamber 26 fuses with the burette 12, therefore being an intrinsic part of the burette itself 12; from the small drip chamber 26 parts a flexible tube sector (which is the equipment) 27 for flow of the liquid contained in the burette 12 towards the patient's vein. This flexible tube sector (equipment) 27 has: a male luer-lock terminal 8 for connection with the needle inserted into the patient's vein (needle not illustrated); a device of "roller clamp" type for flow regulation and a plastic clamp 10 to be triggered when full flow closure is desired.

In figure 3, a syringe 28 is also illustrated schematically with metal needle 29 and a dashed line from the metal needle 29 to the perforable membrane 20 of the burette 12, where the medicine is introduced to that burette 12.

Figure 4 illustrates a schematic view of the coupling of the traditional equipment with burette 11 in a traditional plastic bag for intravenous solutions 1.

Having described the figures, it will now be seen how the traditional procedure for preparing and administering an intravenous solution looks like.

For access to an intravenous solution packaged in a traditional plastic bag for intravenous solutions 1, it is used traditional infusion equipment 4 with plastic spike 5. Access to the intravenous solution is made through the perforation of the membrane of the traditional plastic bag for intravenous solutions 1 by the plastic spike 5 of the traditional infusion equipment 4 (figures 1 and 2) and the other end of traditional infusion equipment 4 exits toward the patient's vein. Thus, the solution to the patient is infused.

If during this intravenous infusion, it is necessary to intermittently add a new medicine to the patient, this will be done by putting this medicine in burette 12 of the traditional equipment with burette 11, as illustrated in the figure 3: the medicine is inserted through a syringe 28, piercing with the metal needle 29 a membrane 20 of this burette 12; to dilute the intermittent medication contained in the burette 12, as can be seen in the figure 4, the burette 12 is coupled to the traditional plastic bag for intravenous solutions 1 containing the desired diluent, by piercing a membrane of said bag 1 of diluent for a plastic spike 5 that exists at the end of the plastic tube sector 23 coming out of the top of the burette 12. From the lower end of burette 12 a plastic tube sector comes out (equipment) 27 towards the patient's vein.

Therefore, to administer the medicine that is in the burette 12, after this preparation described above, the set illustrated in figure 2 is taken from the patient e the traditional equipment with burette 11, illustrated in figure 4, is inserted.

After this intermittent infusion is completed, the set shown in figure 4 is disconnected from the patient and the set shown in figure 2 is reconnected to the patient.

### PROBLEMS OF THE STATE OF THE ART

Given the traditional procedures performed for the preparation and administration of pharmaceutical solutions by intravenous infusion, using syringe 28 with metal needle 29, traditional plastic bag for intravenous solutions 1 and traditional equipment with burette 11, all belonging to the state of the art, it remains easy to understand that these membrane puncture procedures (by metal needles 29 or by plastic spikes 5) can generate particles, that contaminate solutions, besides exposing the professional to risks of puncture accidents and the patient to risks of particle embolization and contamination, when these connection-disconnection procedures for bags, equipment and vein access are repeated.

In addition to the various risk situations involved in this traditional procedure for preparing and administering pharmaceutical solutions by intravenous infusion, it should be noted the complexity and time spent in this obsolete procedure which uses, particularly, the traditional equipment with burette 11, dependent on metal needles 29 and plastic spikes 5.

### OBJECTS OF THE INVENTION PATENT

In view of the drawbacks noted in the above-mentioned state of the art, it is an object of this Invention Patent application the object defined as a dilution and drip chamber using a valve with open-close mechanism, addressed in the Invention Patent application BR 10 2016 016091 0, which is an enhancement of the analog device in the plastic bag for intravenous solutions addressed in the Invention Patent already granted PI 1003460-9, commercially identified by the trademark "NC Bag®".

This dilution and drip chamber, which is the object of this Invention Patent application, will be used in conjunction with this bag (commercially identified by the trademark "NC Bag®") and with thee infusion equipment with luer-lock terminal, addressed in the granted Invention Patent PI1003460-9 and Invention Patent application BR 10 2016 016091-0, as with luer-lock syringe without needle.

The operations performed with this new dilution and drip chamber, both in the preparation of injectable solutions and the administration of intravenous solutions, are done without the use of metal needles and plastic spikes, using its luer-lock connections (male and female luer-lock terminals) and also the luer-lock connections of the devices mentioned in the previous paragraph.

It is further an object of this Invention Patent application to provide a dilution and drip chamber which results in a significant simplification of the procedures necessary for the preparation and administration of injectable solutions, reducing occupational risks of puncture accidents, solution contamination and particle embolization.

### BRIEF DESCRIPTION OF THE INVENTION

The dilution and drip chamber, which is the object of this Invention Patent application, is intended for dilution of injectable solutions and functions as a drip chamber for intravenous infusion of these solutions, designed for use in conjunction with a plastic bag for intravenous solutions, with valve with innovative open-and-close mechanism, commercially identified by the trademark "NC Bag®", and with an infusion equipment with male luer-lock terminal (without plastic piercing), addressed in the granted Invention Patent PI1003460-9 and Invention Patent application BR 10 2016 016091-0, and also using syringe with male luer-lock terminal without needle.

The dilution and drip chamber, object of this Invention Patent application, as well as the other devices cited, allows safe, fast handling without particle generation and substantially reduces the possibility of contamination when preparing injectable drug solutions.

The dilution and drip chamber proposed herein has its structure defined by a plastic tube sector (burette), closed top and bottom by its top and bottom covers. There is in the top cover, in addition to an air inlet with filter, a tubular extension, at the end of which is incorporated a male luer-lock terminal that allows the dilution and drip chamber presented herein to be directly coupled to a plastic bag for intravenous solutions endowed with valve with open-and-close mechanism, as addressed in the granted Patent Document (PI1003460-9), owned by the applicant, as well as in the Invention Patent application (BR 10 2016 016091-0) also owned by the applicant. The bottom cover incorporates a tubular extension, at the end of which a valve with open-and-close mechanism is aggregated, as discussed in the aforementioned granted Patent Document PI1003460-9, as well as in the Invention Patent application (BR 10 2016 016091-0). This valve with open-and-close mechanism has a female luer-lock terminal where can be coupled an infusion equipment, endowed with male luer-lock terminal, as addressed in the granted Patent Document PI1003460-9, as well as in the Invention Patent application also owned by the applicant (BR 10 2016 016091-0), or coupled to a syringe, without metal needle, and endowed with male luer-lock terminal. The dilution and drip chamber has a cover to close its male luer-lock terminal; this same cover can be used for closing the male luer-lock terminal of the infusion equipment or for closing the male luer-lock terminal of the syringe.

### DESCRIPTION OF THE FIGURES

The object of this Invention Patent application will be fully described and understood in its entirety from the following related figures, in which:
Figure 1 illustrates a general and schematic view of a traditional plastic bag for intravenous solutions and an infusion equipment (partially pictured), both belonging to the state of the art, whereas in said view the infusion equipment is depicted at a stage prior to its coupling to the output port of the traditional plastic bag for intravenous solutions.
Figure 2 illustrates a general and schematic view of the traditional plastic bag for intravenous solutions, belonging to the state of the art, depicted in figure 1, the traditional infusion equipment being coupled to its output port, also belonging to the state of the art, this traditional infusion equipment being fully illustrated.
Figure 3 illustrates a perspective view of a typical example of a traditional equipment with burette, pictured in its entirety; the view also includes an equally schematic representation of a syringe endowed with metal needle; a dashed line indicates the insertion point of the metal needle into the perforable membrane of the fluid inlet of the burette.
Figure 4 illustrates a perspective view of the coupling of the traditional equipment with burette in a traditional plastic bag for intravenous solutions, both belonging to the state of the art.

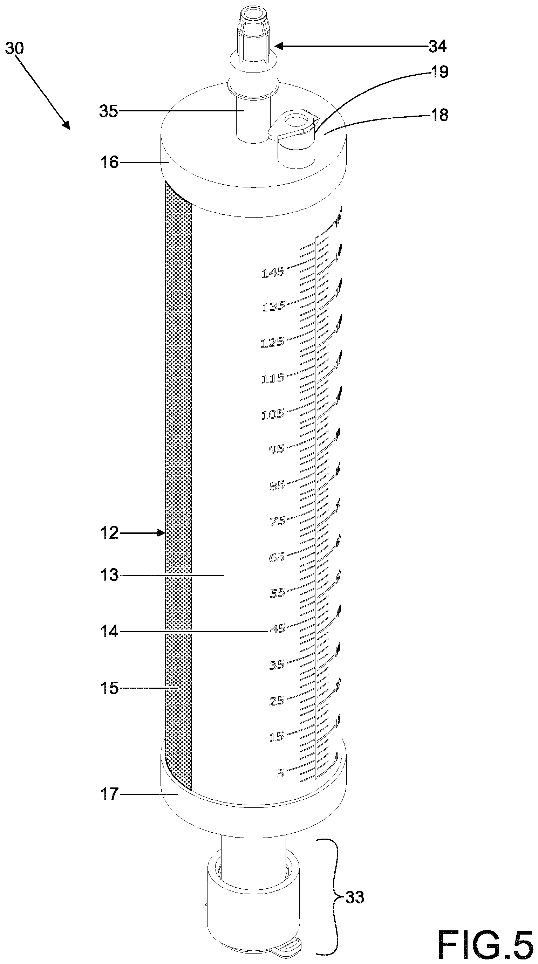

Figure 5 illustrates a general and top perspective view of the dilution and drip chamber, object of this Invention Patent application.

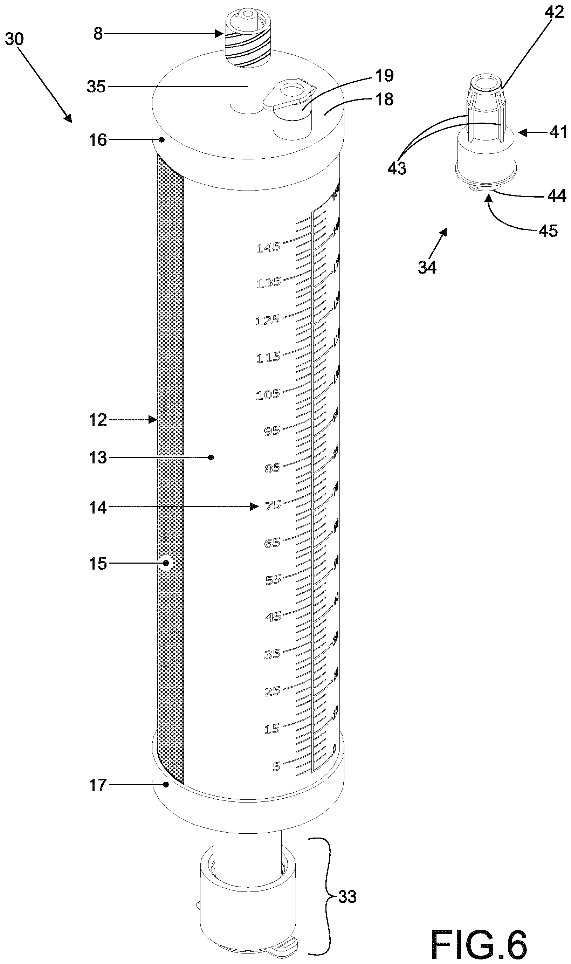

Figure 6 illustrates a general and top perspective view of the dilution and drip chamber, object of this Invention Patent application, pictured without its cover, which is illustrated separately beside.

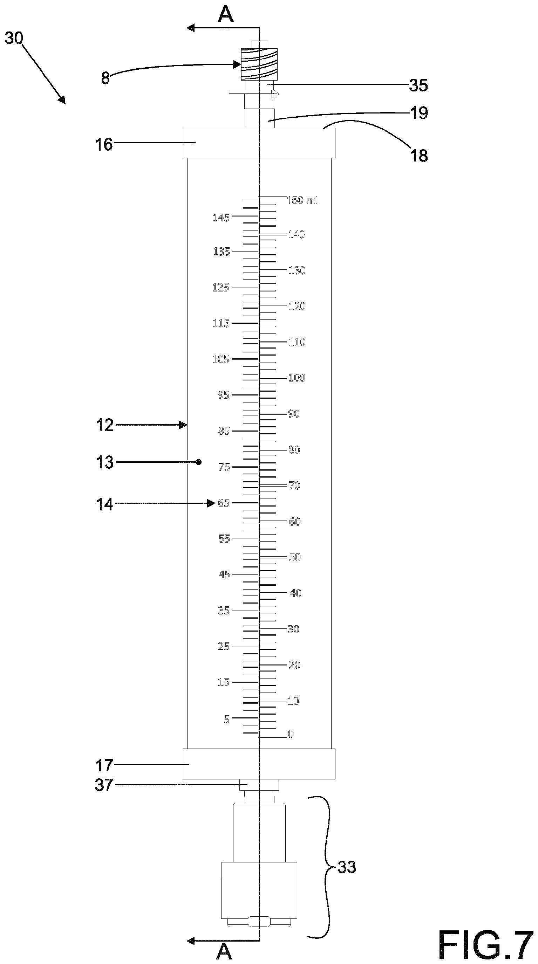

Figure 7 illustrates a frontal and general view of the dilution and drip chamber, object of this Invention Patent application.

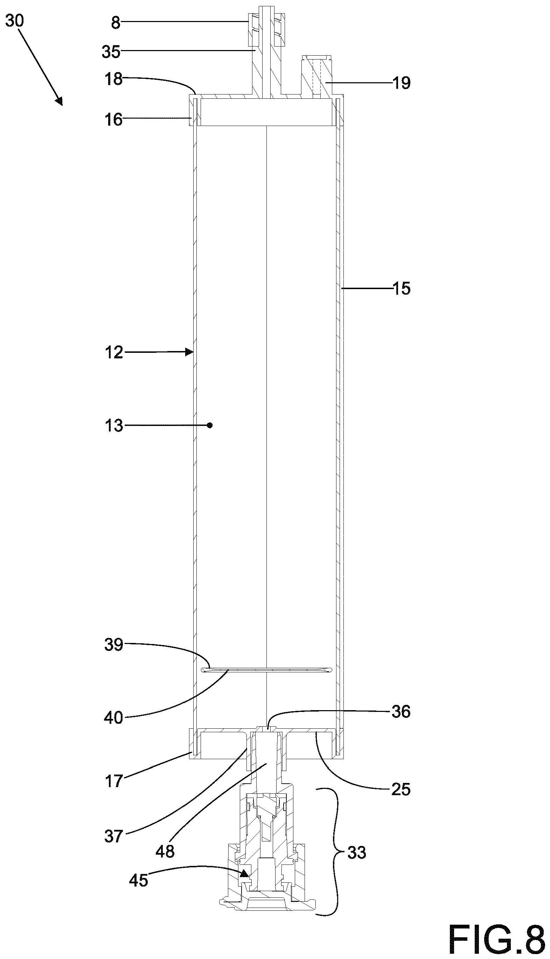

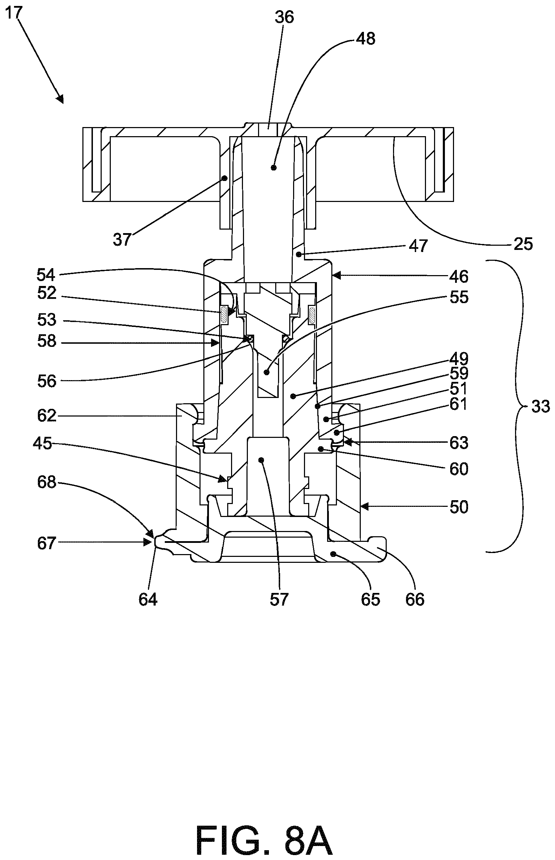

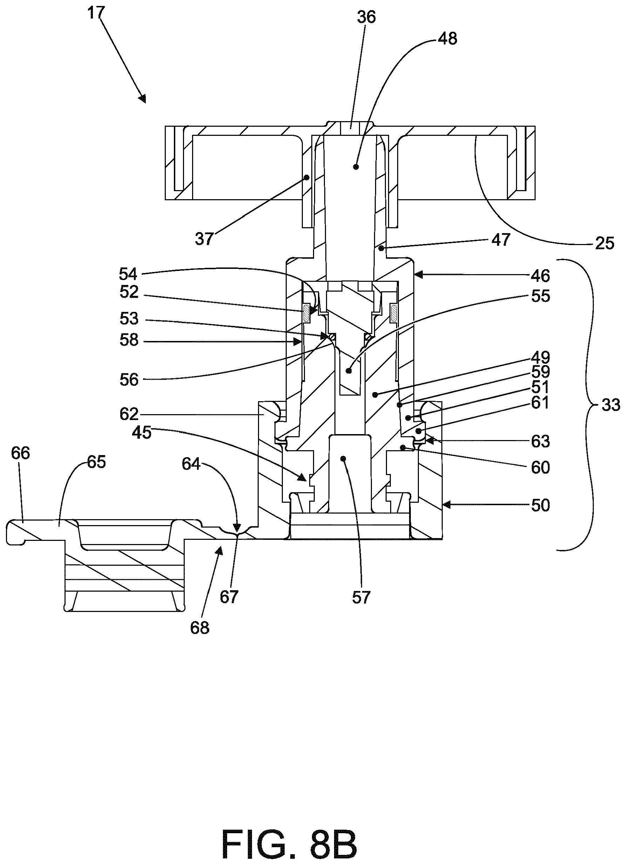

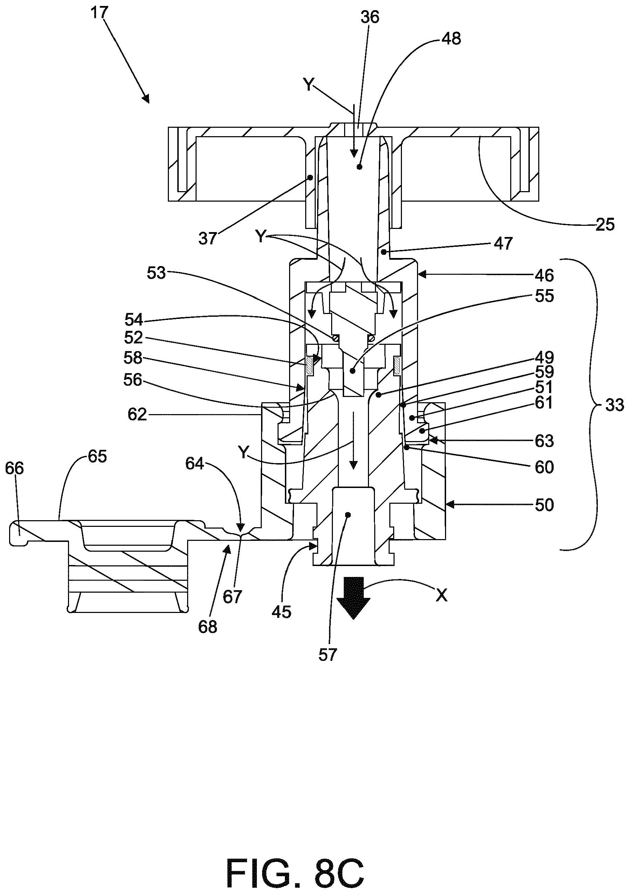

Figure 8 illustrates the dilution and drip chamber, object of this Invention Patent application, according to the cross section "A"-"A" indicated in figure 7; figure 8 also illustrates a section of a floating shutter element (floating membrane) inside the interior of the dilution and drip chamber, wherein said floating shutter element belongs to the state of the art.
Figure 8A illustrates, in cross-section, an isolated view of bottom cover of the dilution and drip chamber, where the valve with open-and-close mechanism is connected, as addressed in the granted Invention Patent PI1003460-9 and Invention Patent application BR 10 2016 016091-0; the valve with open-and-close mechanism is with its cover in the closed position.
Figure 8B illustrates a view similar to that depicted in figure 8A, where the valve with open-and-close mechanism is with its cover in the open position and with the valve mechanism closed, thus blocking the flow of liquid.
Figure 8C illustrates a view similar to those depicted in figures 8A and 8B, where the valve with open-and-close mechanism is with its cover in the open position and the open-and-close mechanism is also opened, allowing the liquid flow.

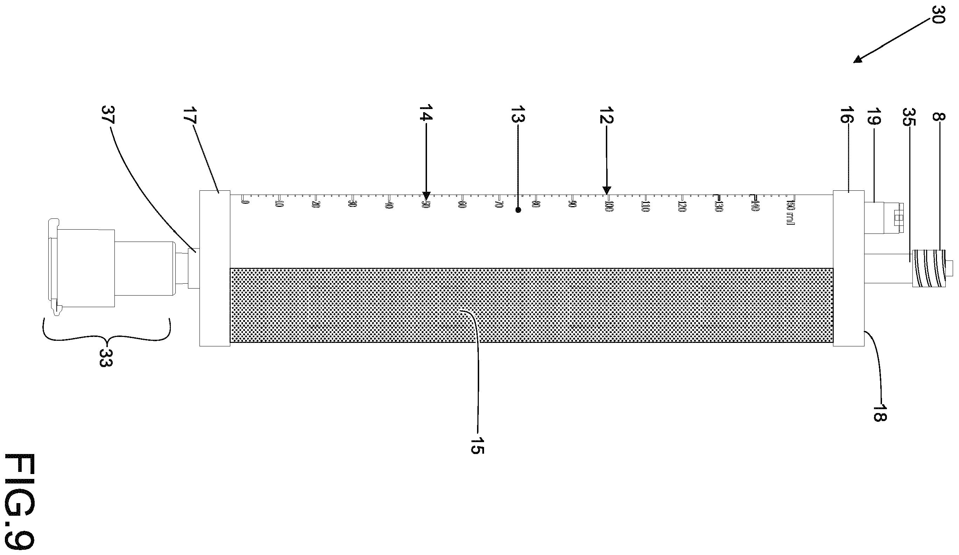

Figure 9 illustrates a side view of the dilution and drip chamber, object of this Invention Patent application.

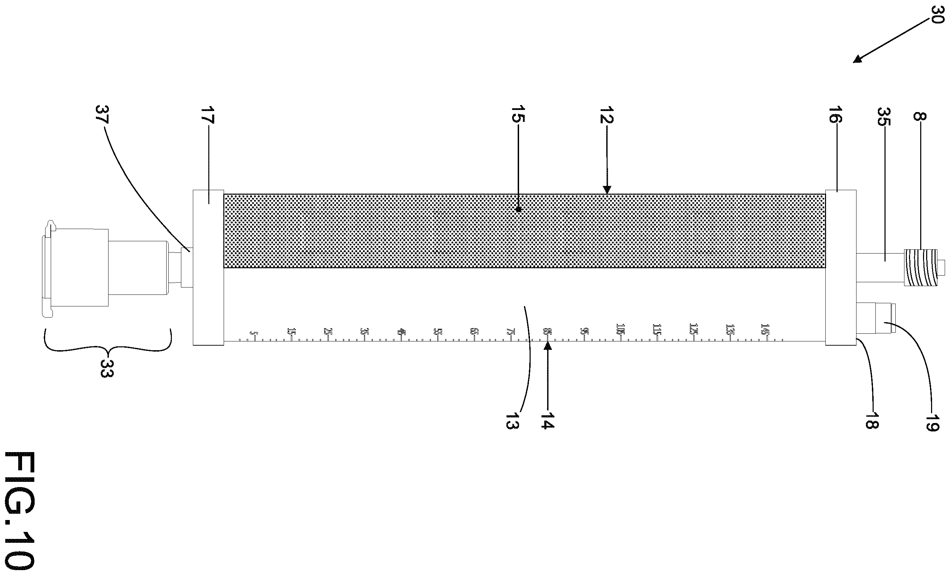

Figure 10 illustrates a view of the opposite side of the dilution and drip chamber, object of this Invention Patent application.

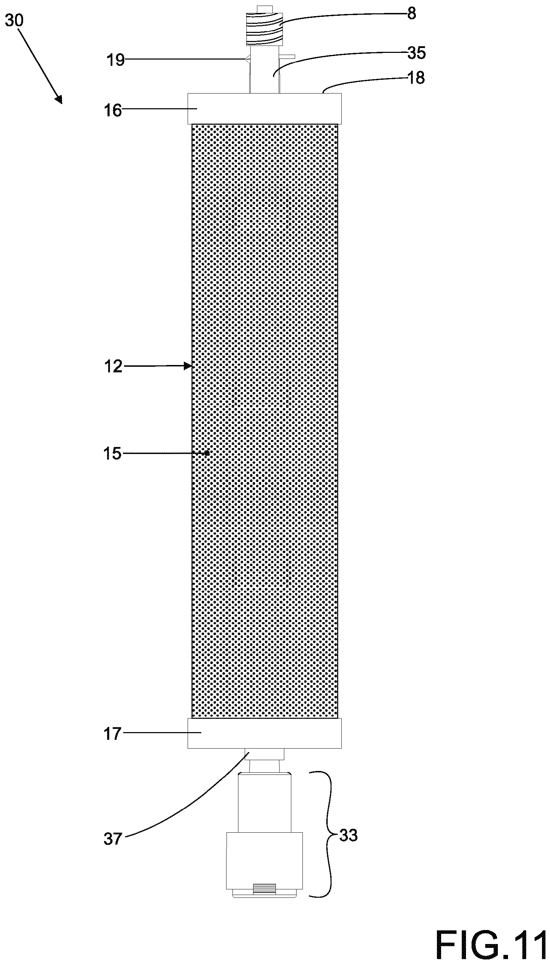

Figure 11 illustrates a rear view of the dilution and drip chamber, object of this Invention Patent application.

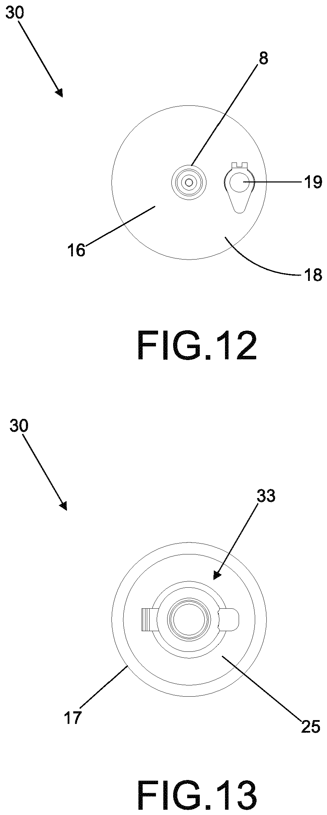

Figure 12 illustrates a top view of the dilution and drip chamber, object of this Invention Patent application.
Figure 13 illustrates a bottom view of the dilution and drip chamber, object of this Invention Patent application.

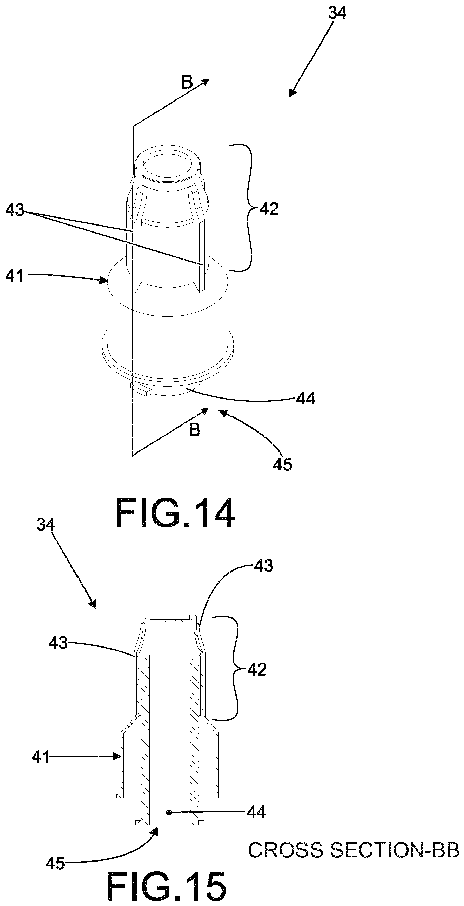

Figure 14 illustrates an isolated perspective view of the cover incorporating the dilution and drip chamber, object of this Invention Patent application; this cover also features compatibility for use in both luer-lock connection of the infusion equipment, addressed in the granted Invention Patent application PI1003460-9 and Invention Patent application BR 10 2016 016091-0, and in the luer-lock terminal of a syringe belonging to the state of the art.
Figure 15 illustrates a full sectional view of the cover of the dilution and drip chamber, object of this Invention Patent application, according to the cross section "B"-"B" indicated in figure 14.

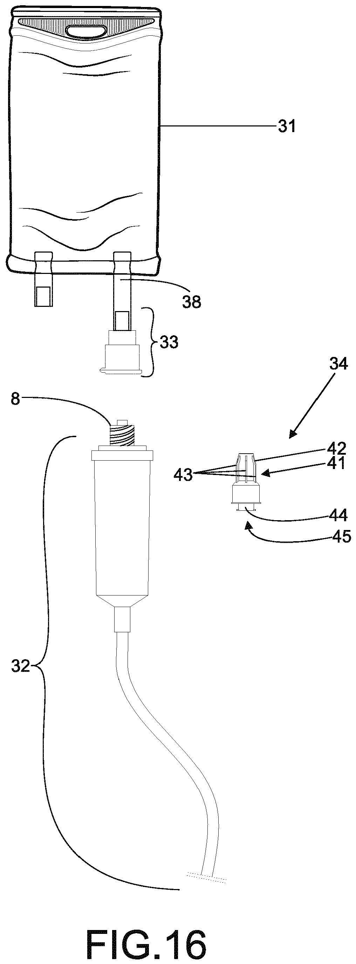

Figure 16 illustrates, schematically, an overview of a model of plastic bag for intravenous solutions, and of an infusion equipment, as addressed in the granted Invention Patent PI1003460-9 and Invention Patent application BR 10 2016 016091-0; the infusion equipment can be closed with the same type of cover integrating the dilution and drip chamber, object of this Invention Patent application.

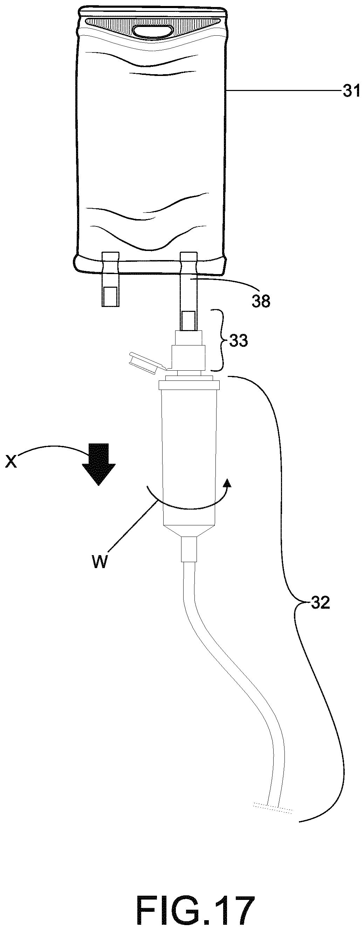

Figure 17 illustrates a plastic bag for intravenous solutions and the infusion equipment, illustrated in figure 16, properly coupled to each other.

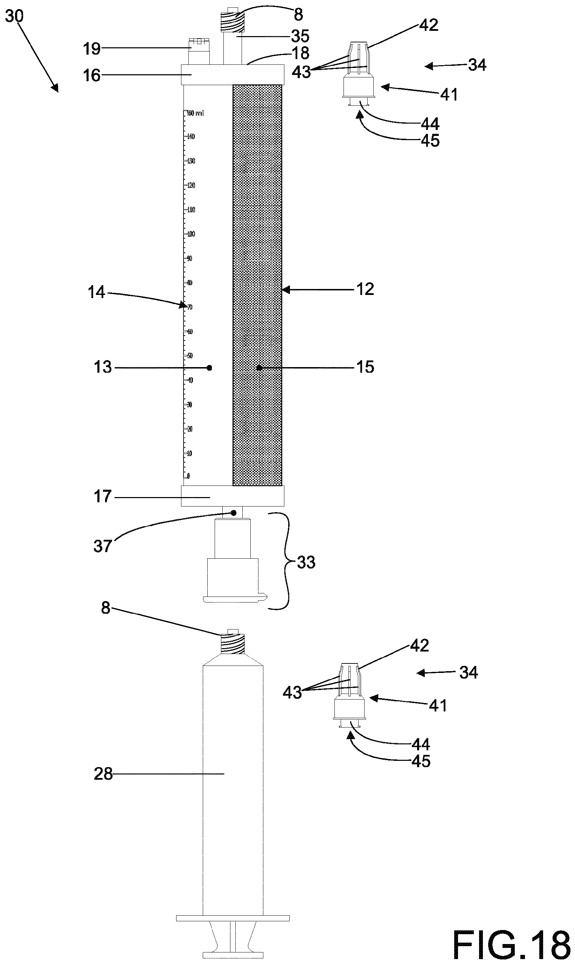

Figure 18 illustrates an overview of the dilution and drip chamber, object of this Invention Patent application, positioned relative to a luer-lock syringe, devoid of metal needle; said view further illustrates two copies of the cover that equips the dilution and drip chamber, one intended for dilution and drip chamber itself and the other to close the luer-lock terminal of the syringe.

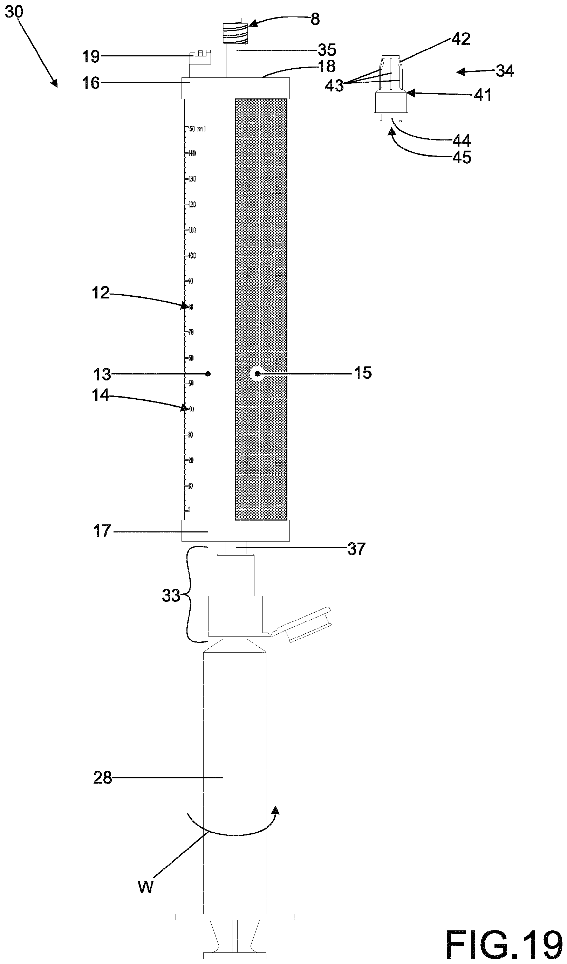

Figure 19 illustrates a side view of the dilution and drip chamber, object of this Invention Patent application; a luer-lock syringe (devoid of metal needle) is connected to the valve with open-and-close mechanism, as addressed in the granted Patent Document PI1003460-9, as well as in the Invention Patent application BR 10 2016 016091-0; figure 19 also shows, separately, the cover that equips the dilution and drip chamber.

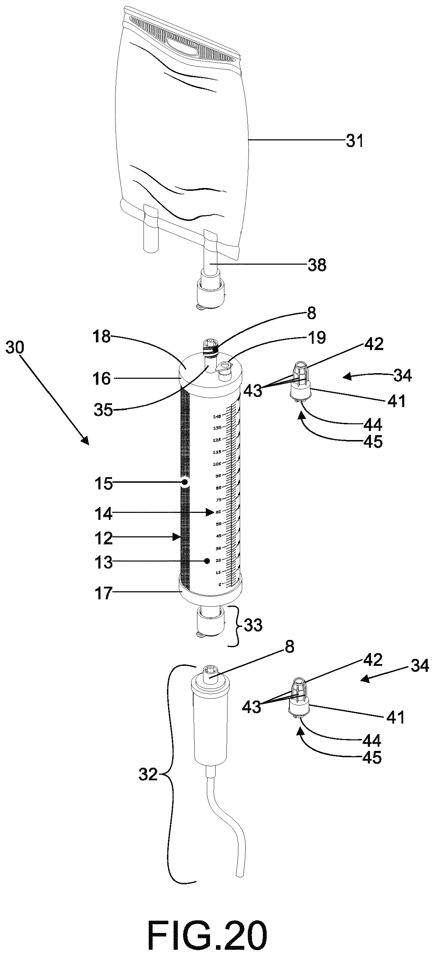

Figure 20 illustrates a view showing sequentially and separately a plastic bag for intravenous solutions, as addressed in the granted Invention Patent PI1003460-9 and Invention Patent application BR 10 2016 016091-0; an example of the dilution and drip chamber, object of this Invention Patent application, and its respective cover; and an example of the infusion equipment, as addressed in the granted Invention Patent PI1003460-9 and Invention Patent application BR 10 2016 016091-0, that can be closed with the same copy of the cover of the dilution and drip chamber.

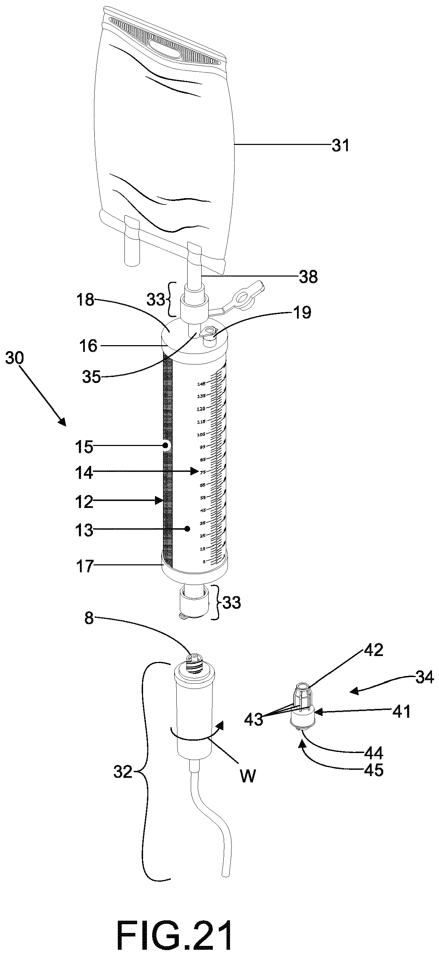

Figure 21 illustrates the dilution and drip chamber properly coupled to the plastic bag for intravenous solutions, as addressed in the granted Invention Patent PI1003460-9 and Invention Patent application BR 10 2016 016091-0; the infusion equipment, as addressed in the granted Invention Patent PI1003460-9 and Invention Patent application BR 10 2016 016091-0, is illustrated herein under non-coupling condition with the dilution and drip chamber; figure 21 separately illustrates and example of the same closing cover of the dilution and drip chamber, used herein in the closing of the male luer-lock terminal of the infusion equipment.

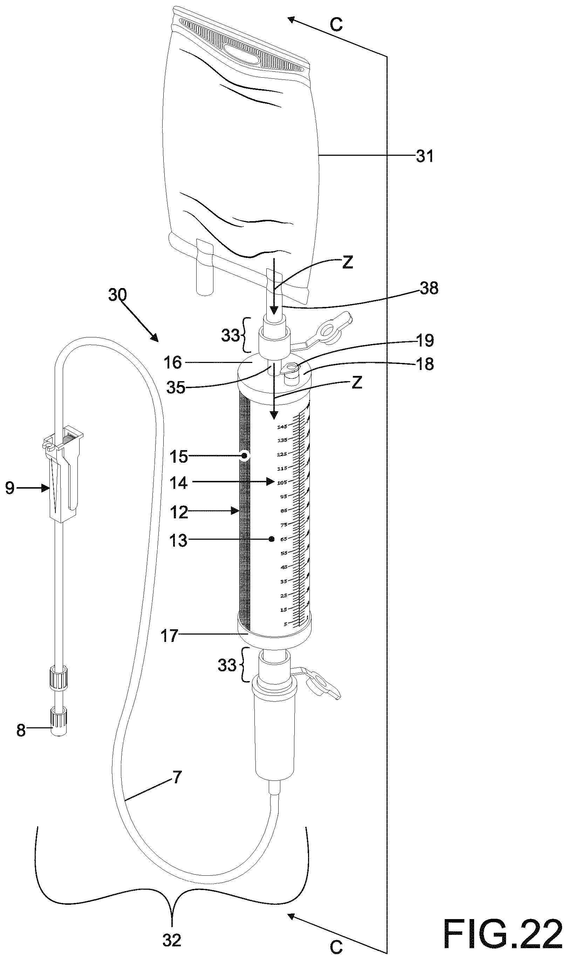

Figure 22 illustrates and overview of the dilution and drip chamber properly coupled to a plastic bag for intravenous solutions, addressed in the granted Invention Patent PI1003460-9 and Invention Patent application BR 10 2016 016091-0, and to an infusion equipment with male luer-lock terminal, equally addressed in the granted Invention Patent PI1003460-9 and Invention Patent application BR 10 2016 016091-0.

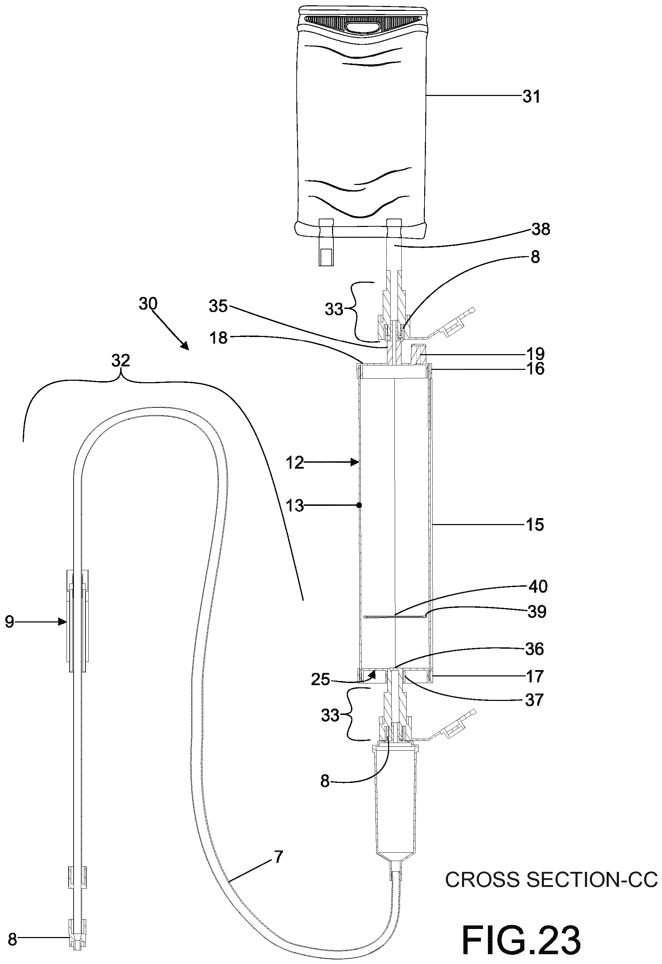

Figure 23 illustrates the assembly of the dilution and drip chamber, plastic bag for intravenous solutions e infusion equipment, according to the cross section "C"-"C" indicated in figure 22.

### DETAILED DESCRIPTION OF THE INVENTION

Preliminarily, through figures 1 a 4, in the topic STATE OF THE ART of this specification, the devices belonging to the state of the art and public domain, have been described, which are traditionally used for the preparation and administration of solutions by intravenous infusion: traditional plastic bag for intravenous solutions 1, traditional infusion equipment 4 and traditional equipment with burette 11.

After this preliminary description, a detailed description of the dilution and drip chamber 30, object of this Invention Patent application, will be presented.

As mentioned before, the dilution and drip chamber 30, object of this Invention Patent application, could only be conceived by incorporating the concepts and devices developed in the granted Invention Patent PI1003460-9 and also in the enhancement that prompted the Invention Patent application (BR 10 2016 016091-0) of the "Válvula com mecanismo abre-e-fecha para bolsa utilizada para acondicionamento, reconstituição e/ou diluição de produtos injetaveis".

The dilution and drip chamber 30, object of this Invention Patent application, is not intended for use with traditional plastic bags for intravenous solutions 1 nor with the traditional equipment of intravenous infusion 4; it is designed to provide a safe means for the preparation and administration of injectable pharmaceutical solutions, in conjunction with plastic bag for intravenous solutions 31 with valve with open-and-close mechanism 33 and infusion equipment 32 with male luer-lock terminal 8, addressed in the granted Invention Patent PI1003460-9 and Invention Patent application BR 10 2016 016091-0.

The set, composed of plastic bag for intravenous solutions 31 with valve with open-and-close mechanism 33, infusion equipment 32 with male luer-lock terminal 8, dilution and drip chamber 30 with valve with open-and-close mechanism 33 and male luer-lock terminal 8 and syringe 28 without metal needle 29, represents the modern approach to preparing and administering injectable solutions, revolutionizing the procedures. This is not an expression of desire but reality, considering that the valve has already been industrially finished and incorporated into solution bags, that have already undergone laboratory stability processes and practical tests in referral hospitals. The dilution and drip chamber, object of this Invention Patent application, integrates the same operational concept, complementing safe and fast devices for the preparation and administration procedures of injectable pharmaceutical solutions.

This set will thus ensure procedural safety, simplicity of execution and time savings, revolutionizing the preparation and administration operations of these solutions, being the ideal answer to rationalize the hospital areas of preparation of medicines and, particularly, to respond safely and quickly to emergency demands in ambulances, in areas of tragedy and in areas of armed conflict. This set of operations will also enable the pharmaceutical industry to be revolutionized by using modern filling devices which will be coupled to this set and which will merit further Invention Patent applications as they cannot be described in this application.

The figures of the Invention Patent application proposed herein, taken according to the professional look of those who transit in this technical area, easily demonstrate what this set represents in terms of this mentioned revolution of procedures. Elimination of membrane piercing connections by plastic spikes and elimination of the metal needle will do more for medicine, prophylaxis of puncture accidents and prophylaxis of contamination, which all campaigns have never done.

Figures 5 to 8 and 9 to 13 illustrate views of dilution and drip chamber 30, object of this Invention Patent application.

Figures 8A, 8B e 8C illustrate, in isolation, the bottom cover 17 of the dilution and drip chamber 30 and its association with the valve with open-and-close mechanism 33.

Figures 14 and 15 illustrate in isolation the cover 34, which is integral part of the dilution and drip chamber 30, for closing the male luer-lock terminal 8 of this chamber, such as, for example, illustrated in figure 8A; this cover 34 was projected to also close the male luer-lock terminal 8 do infusion equipment 32 (as seen in figure 16), addressed in the granted Invention Patent PI1003460-9 and Invention Patent application BR 10 2016 016091-0, e also the male luer-lock terminal 8 of syringe 28 (as seen in figure 18), thus avoiding a profusion of cover designs generating confusion when working with these medical devices.

Figures 16 to 23 illustrate the possible connections among the components of this new set for preparation and administration of injectable solutions.

The dilution and drip chamber 30, as can be seen in the figure 5, comprises a transparent and semi-rigid plastic tube sector 12 that, in the frontal portion of its wall 13, presents a graduated volume scale 14 (expressed in ml), and in the posterior portion of the wall 13 presents finishing in white background 15, aiming to facilitate the visualization of the graduated volume scale 14 and the verification of any problems in the solution such as, for example, particles.

As the dilution and drip chamber 30 is made of semi-rigid plastic (that did not self-draw), it meets the technical standards for intravenous infusion devices, incorporating a float ring 39 with a thin central membrane 40 and an air filter 19, belonging to the state of the art, which will be described below.

In figure 5, it is observed that the extremes of the transparent and semi-rigid plastic tube sector 12 of the dilution and drip chamber 30 are closed for a top cover 16 and for a bottom cover 17. In face 18 of top cover 16 there is an air filter 19 with cover and in the central region a tubular extension 35 with a male luer-lock terminal 8, which in this figure 5 is illustrated closed for a cover 34.

As can be seen in figures 8 and 23, in the inner face of bottom cover 17 there is a liquid through hole 36 that goes towards valve with open-and-close mechanism 33. This valve 33 incorporates into the dilution and drip chamber 30 through a tubular extension 37 which parts of the base wall 25 of the bottom cover 17 of this dilution and drip chamber 30.

This valve with open-and-close mechanism 33 of the dilution and drip chamber 30 is the same device of the outlet port 38 of the plastic bag for intravenous solutions 31 implemented according to what addressed in the granted Invention Patent PI 1003460-9 and the Invention Patent application BR 10 2016 016091 0, as can be observed in figures 16, 17, 20, 21, 22 e 23.

Thus, despite the valve with open-and-close mechanism 33 does not constitute itself, and in isolation, the claimed object in this Invention Patent application, it is what allows the realization of this dilution and drip chamber 30, object of this Invention Patent application, transforming this dilution and drip chamber 30 into an innovative product, with all characteristics to revolutionize the way the injectable pharmaceutical solutions are prepared and administered.

By technical requirement, according to figures 8 and 23, the dilution and drip chamber 30 also incorporates a floating ring 39 with a thin central membrane 40 to, once drained the entire solution of the dilution and drip chamber 30, close the liquid through hole 36, avoiding the air passage towards the patient's vein.

The cover 34, which integrates the dilution and drip chamber 30, object of this Invention Patent application, as highlighted in the figures 14 and 15, has an outer body 41 where, in its top portion 42 there are radial ribs 43 to allow ease of handling at the time of its application (by rotational movement from left to right) and at the time of withdrawal (by rotational movement from right to left). In the central part of the cover 34 there is a hollow plastic tube 44 protruding toward the base of the cover but extending slightly beyond that base and ending in the format of female luer-lock terminal 45. As can be seen in the picture 6, this female luer-lock terminal 45 of the cover 34 will close the male luer-lock terminal 8 of the dilution and drip chamber 30.

This cover model 34 can also close the male luer-lock terminal 8 of the infusion equipment 32 or the male luer-lock terminal 8 of the syringe 28.

This cover model 34 serves, however, to avoid a profusion of covers on pharmaceutical preparation tables. Its design encourages the professional to keep the cover 34 always connected to some device or lying on the work table and not supported vertically; when lying down, the outer body 41 protects the female luer-lock terminal 45 which will be connected to male luer-lock terminals 8 of the previously mentioned medical devices.

The infusion equipment 32, which is illustrated in full in figures 22 and 23, has at the end of the hose 7 a male luer-lock terminal 8 for connection with the needle of the patient's vein, and a roller clamp 9 to control the liquid flow.

Figures 8A, 8B and 8C illustrate, in isolation, the bottom cover 17 of the dilution and drip chamber 30 and its association with the valve with open-and-close mechanism 33.

As can be seen in figure 8A, the valve with open-and-close mechanism 33 has a conical base 46 that suffers a narrowing defining a tubular terminal 47 that fits into the tubular extension 37 of the bottom cover 17 of the dilution and drip chamber 30. The liquid from the semi-rigid and transparent plastic tube sector 12 illustrated in figure 8 passes through the through hole 36 to the inner channel 48 of the valve 33.

In the conical base 46, a mobile conical part is inserted 49 which presents a female luer-lock terminal 45.

The cover 50 fits to the final part 51 of the conical base 46 to protect the female luer-lock terminal 45 of the mobile conical part 49 and also prevent this part from disconnecting from valve with open-and-close mechanism 33.

The valve with open-and-close mechanism 33 has two O ring sealing rings, being one of larger diameter 52 and the other of smaller diameter 53.

The larger diameter sealing ring 52 is mounted in the groove 54 of the mobile conical part 49 establishing the lateral seal between said mobile conical part 49 and the conical base 46, whereas the smaller diameter sealing ring 53, which is mounted at the bottom of the core 55 of the conical base 46, ensures central obstruction of the flow of liquid through the valve with open-and-close mechanism 33 when it is in its closed condition.

When the valve with open-and-close mechanism 33 is totally closed, the smaller diameter sealing ring 53 maintains contact with the diameter reduction region 56 members of the axial channel 57 of the mobile conical part 49. The surface 58 of the mobile conical part 49 keeps contact the inner surface 59 of the conical base 46.

The mobile conical part 49 has an annular edge 60 that maintains contact with the ring edge 61, which belongs to the conical base 46, whenever the valve with open-and-close mechanism 33 is closed.

The plastic cover 50 has a tubular portion 62 which, in its inner face, has an annular groove um 63, which fits the annular edge 61 of the conical base 46, to secure the cover 50 to said conical base 46.

The plastic cover 50 has a flap 64 that connects its tubular portion 62 to the closing portion 65 which contains a radial flap 66 that protrudes from the closing portion 65. The flap 64 is folded in its middle region 67, thereby causing this tab 64 to act like a hinge 68 allowing the closing portion 65 to be angularly displaced from a closing position, as illustrated in the figure 8A, to an open position, as illustrated in the figures 8B and 8C.

When the cover 50 is closed, the female luer-lock terminal 45 inside is fully protected, whereas when said cover 50 is open, the female luer-lock terminal 45 can be accessed to attach an infusion equipment 32 with male luer-lock terminal 8 (as illustrated in figures 16, 17, 20, 21, 22 and 23), or a syringe 28 with male luer-lock terminal 8, without metal needle 29 (as illustrated in figures 18 and 19).

Connected to the male luer-lock terminal 8 of the infusion equipment 32 or the male luer-lock terminal 8 of syringe 28 to the female luer-lock terminal 45 of the valve with open-and-close mechanism 33, a traction movement in the equipment or in the syringe (indicated by the arrow "X" in figures 8C and 17) displace the mobile conical part 49 of the valve with open-and-close mechanism 33 allowing the liquid release; the opposite movement indicated by the arrow "X" closes the valve with open-and-close mechanism 33.

In figure 8C, the arrows "Y" indicate the liquid flow that runs through valve with open-and-close mechanism 33.

The valve with open-and-close mechanism 33 incorporated to the dilution and drip chamber 30 is the same valve with open-and-close mechanism 33 which integrates the plastic bag for intravenous solutions 31, the opening mechanism of which has been previously described. However, the valve with open-and-close mechanism 33 of the dilution and drip chamber 30 also opens by traction exerted on the infusion equipment 32 or in the syringe 28 coupled to it; the opposite movement closes the valve with open-and-close mechanism 33.

In figure 22, the arrows "Z" indicate the liquid flow, contained within the plastic bag for intravenous solutions 31, for the dilution and drip chamber 30.

The dilution and drip chamber 30 is thus intended for dilution of injectable solutions and acts as a drip chamber for intravenous infusion of these solutions, having been designed as already mentioned, for use in conjunction with plastic bag for intravenous solutions 31, with valve with open-and-close mechanism 33, commercially identified by the trademark "NC Bag®", and with the infusion equipment 32 with male luer-lock terminal 8, addressed in the granted Invention Patent PI1003460-9 and Invention Patent application BR 10 2016 016091-0, and, yet, using syringe 28 with male luer-lock terminal 8, without metal needle 29.

A second, quite practical possibility is to use dilution and drip chamber 30 for preparing injectable medications, introducing and removing multiples doses of products through a syringe 28 with male luer-lock terminal 8, without metal needle 29, through valve with open-and-close mechanism 33 of the dilution and drip chamber 30.

Using the described set, the operation of which will be shown below, gains in safety, speed and practicality are evident.

To administer an intravenous solution contained in a plastic bag for intravenous solutions 31, commercially identified by the trademark "NC Bag®" (figure 16), it is used the infusion equipment 32, with male luer-lock terminal 8. The infusion equipment 32 is connected (figure 17) to the plastic bag for intravenous solutions (commercially identified by the trademark "NC Bag®") 31, through the rotational movement from left to right (arrow "W"). Pulling the dripper from the infusion equipment 32, the valve with open-and-close mechanism 33, incorporated into plastic bag for intravenous solutions 31, is open, allowing the intravenous solution to pass through, that will reach the patient from the lower end of the infusion equipment 32.

If intermittent administration of another medication to the patient receiving this described solution is required, this will be done using the new dilution and drip chamber 30 (figure 5), equipped with valve with open-and-close mechanism 33, without the presence of perforating membranes, object of this Invention Patent application.

According to figure 17, the intermittent medication is inserted into the dilution and drip chamber 30 with syringe 28 with male luer-lock terminal 8, without metal needle 29. The syringe 28 will be coupled to the valve with open-and-close through rotational movement from left to right (arrow "W"). By pulling the syringe 28, the valve with open-and-close mechanism 33 of the dilution and drip chamber 30 is open, allowing the insertion of intermittent medication.

To dilute the intermittent medication contained in dilution and drip chamber 30, this device is coupled to a plastic bag for intravenous solutions 31 (commercially identified by the trademark "NC Bag®") (figure 21) where is the diluent to be used. The male luer-lock terminal 8 of the dilution and drip chamber 30 is connected to the female luer-lock terminal 45 of the valve with open-and-close mechanism 33 of the plastic bag for intravenous solutions 31 through the rotational movement from left to right (arrow "W"); pulling the body of the dilution and drip chamber 30, the valve with open-and-close mechanism 33 is open to allow the passage of the diluent to the dilution and drip chamber 30 the desired volume (the chamber has degree in ml); thus the medicine is diluted to the desired volume.

To administer the intermittent medication contained in dilution and drip chamber 30, the lower end of this device is connected to the infusion equipment 32 with male luer-lock terminal 8 (figures 22 and 23), through rotational movement from left to right (arrow "W"); pulling the body of the infusion equipment 32, the valve with open-and-close mechanism 33 is open integrated to the dilution and drip chamber 30 allowing the solution to flow to the patient's vein.

In this set, for administration of intermittent medication, the same infusion equipment 32 is used, which was used to administer the previous solution to the patient, without disconnection of the equipment that is in the vein, which avoids further handling with risks of contamination.

The second possibility of dilution and drip chamber 30, in situations where multiple doses of the same product are required for different patients, is to use it for the preparation of injectable medications. A concentrated solution may be introduced into the dilution and drip chamber 30 with a syringe 28 with male luer-lock terminal 8, without metal needle 29, through the valve with open-and-close mechanism 33 (figures 18 and 19). After dilution to the desired concentration, a syringe 28 can be used with male luer-lock terminal 8, without metal needle 29, to withdraw multiple doses of diluted drug.

The dilution and drip chamber 30, coupled to the already defined set composed of plastic bag for intravenous solutions 31, having a valve with open-and-close mechanism 33 and infusion equipment 32 with male luer-lock terminal 8, replaces, with enormous advantages, the traditional equipment with burette 11 that is coupled to traditional plastic bags for intravenous solutions 1 endowed of perforable membranes, as:
- it eliminates the use of metal needle 29 for introducing solution for injection into it; the solution is transferred by syringe 28 with male luer-lock terminal 8;
- it has no "plastic spike" (plastic piercing) for coupling with the diluent bag;
- it needs no handle 21 for support. The dilution and drip chamber 30 is firmly and directly connected to the plastic bag for intravenous solutions 31, freeing up space for any other necessary patient bags and maintaining better support organization;
- male 8 and female 45 luer-lock connections and the valve with open-and-close mechanism 33 allow ease coupling and uncoupling of the system devices;
- the absence of piercing tips and pierceable membranes prevents the generation of particles during the handling of the dilution and drip chamber 30;
- it is possible to change the dilution and drip chamber 30, without decoupling of the infusion equipment 32 with male luer-lock terminal 8 which will be connected to the patient;
- easy coupling with the plastic bag for intravenous solutions (commercially identified by the trademark "NC Bag®") 31 containing diluent which, in addition to diluting the product contained in dilution and drip chamber 30, also allows to wash the dilution and drip chamber 30 in case of preparation of incompatible substances;
- the infusion equipment 32 with male luer-lock terminal 8 attaches quickly and has its lumen easily washed off by a solution contained in the plastic bag for intravenous solutions 31, when contact with incompatible substances must be avoided;
- the infusion equipment 32 with male luer-lock terminal 8 also allows direct connection to a plastic bag for intravenous solutions (commercially identified by the trademark "NC Bag®") 31 containing diluent to perform the "prime" (the initial filling of infusion equipment with this diluent), avoiding, for example, do the "prime" with an antibiotic e run the risk of drug loss and environmental contamination where this product is prepared, events that may lead to the development of resistant bacterial strains that increase the risk of hospital infections.

## Claims

1. Dilution and drip chamber for procedures of intermittent preparation and administration of pharmaceutical solutions by intravenous infusion; wherein the chamber structure (30) comprises a semi-rigid and transparent plastic tube sector (12), closed on the top by a top cover (16) and on the bottom by a bottom cover (17); the top cover (16) on the surface of its wall (18) incorporates, concentrically, a tubular extension (35) provided with a male luer-lock terminal (8), wherein said top cover (16) also has an air inlet with filter (19); the wall (13) of the semi-rigid and transparent plastic tube sector (12) has a graduated volume scale (14) and opposite to the graduated volume scale (14) the semi-rigid plastic tube (12) has white background (15); the bottom cover (17) shows, concentrically, a tubular extension (37) which part of the base wall (25); the tubular extension (37) receives the conical base (46) of a valve with open-and-close mechanism (33), which has a female luer-lock terminal (45); the dilution and drip chamber (30) has a cover (34) for closing its male luer-lock terminal (8); the cover (34) has an outer body (41) where, in its upper portion (42), there are radial ribs (43); in the central part of the cover (34) there is a hollow plastic tube (44) protruding toward the base of the cover (34), but going a little beyond this base, and ending in female luer-lock terminal format (45).

2. Dilution and drip chamber according to claim 1, wherein the dilution and drip chamber (30) can be coupled by connecting its male luer-lock terminal (8), incorporated in the tubular extension (35) which part from the wall (18) of the top cover (16), to the female luer-lock terminal (45) of the valve with open-and-close mechanism (33) incorporated into the outlet port (38) of the plastic bag for intravenous solutions (31).

3. Dilution and drip chamber according to claim 2, wherein the liquid contained in the plastic bag for intravenous solutions (31) can be directly transferred to the dilution and drip chamber (30) when opening the open-and-close mechanism of the valve with open-and-close mechanism (33).

4. Dilution and drip chamber according to claim 1, wherein the dilution and drip chamber (30) can be coupled to an infusion equipment (32) endowed with male luer-lock terminal (8), upon connection of that male luer-lock terminal (8) of the infusion equipment (32) to the female luer-lock terminal (45) of the valve with open-and-close mechanism (33), incorporated to the tubular extension (37) which part from the base (25) of the bottom cover (17) of the dilution and drip chamber (30).

5. Dilution and drip chamber according to claim 4, wherein the liquid contained inside the dilution and drip chamber (30) can be directly transferred to the infusion equipment (32) when opening the open-and-close mechanism of the valve with open-and-close mechanism (33).

6. Dilution and drip chamber according to claim 1, wherein the dilution and drip chamber (30) can be coupled through the female luer-lock terminal (45) of the valve with open-and-close mechanism (33), incorporated to the tubular extension (37) which parts from the base (25) of the bottom cover (17), to the male luer-lock terminal (8) of the syringe (28).

7. Dilution and drip chamber according to claim 6, wherein the liquid contained inside the dilution and drip chamber (30) can be directly transferred to the syringe (28) or, in opposite direction, from the syringe (28) to the dilution and drip chamber (30) when opening the valve with open-and-close mechanism (33) of the dilution and drip chamber (30).

8. Dilution and drip chamber according to claim 6, wherein the dilution and drip chamber (30), through the valve with open-and-close mechanism (33), allows the preparation of injectable medications in the dilution and drip chamber (30), inserting and removing multiple doses of the products, through a syringe (28) with male luer-lock terminal (8).

9. Dilution and drip chamber according to claim 1, wherein the cover (34) can be employed to close the male luer-lock terminal (8) of the infusion equipment (32) or the syringe (28).
